# EUROPÄISCHE PATENTSCHRIFT

(11) **EP 1 413 320 B1**
(45) Veröffentlichungstag und Bekanntmachung des Hinweises auf die Patenterteilung: **05.07.2006**
(21) Anmeldenummer: 03090347.0
(22) Anmeldetag: 16.10.2003
(51) Int. Cl.: A61L 24/02, A61L 27/12

(54) **Pulvergemisch für resorbierbare Calciumphosphat-Biozemente**
Powder mixture for resorbable calcium phosphate bio-cements
Mélange de poudre pour un ciment résorbable à base de phosphate de calcium

(30) Priorität: 21.10.2002 DE 10249625
(43) Veröffentlichungstag der Anmeldung: 28.04.2004
(73) Patentinhaber: BAM Bundesanstalt für Materialforschung und -prüfung, 12205 Berlin (DE)
(72) Erfinder: Berger, Georg, 16341 Zepernick (DE); Marx, Heidi, 10315 Berln (DE); Jäger, Christian Prof., 07749 Jena (DE); Pauli, Jutta, 12555 Berlin (DE)
(74) Vertreter: Walter, Wolf-Jürgen

(56) Entgegenhaltungen:
- EP-A- 1 153 621
- WO-A-91/07357
- DE-C- 19 744 809
- US-A- 3 922 155

## Beschreibung

Die Erfindung betrifft Pulvergemische auf Basis von Calciumphosphaten. Die Erfindung betrifft auch die Verwendung der Pulvergemische zur Herstellung von Biozementen mit hoher Löslichkeit, die die bislang nach dem Abbinden bekannten Kristallphasen Hydroxylapatit(HA), präzipitierten Apatit (PHA), Calcium-defizienten-Hydroxylapatit(Ca-d-HA) in nur untergeordneten Anteilen, d.h. als Nebenphasen enthalten.

Anorganische Materialien mit hoher Resorbierbarkeit sind an sich bekannt. Auch Werkstoffe, die ihren speziellen Einsatz als bioaktive Knochenersatzwerkstoffe finden und eine schnelle Löslichkeit besitzen, sind in der Literatur beschrieben. Beispielsweise wurde ständig über den erfolgreichen klinischen Einsatz von Keramiken mit den Hauptkristallphasen alpha- oder beta-Tricalciumphosphat (TCP) berichtet. Zudem gab es auch vergleichende Untersuchungen dieser beiden TCP-Phasen im Tierversuch . Für die folgenden Ausführungen ist es von Bedeutung speziell auf ein alpha-TCP hinzuweisen, dass an der Oberfläche daraus hergestellter Granulate Dicalciumphosphat enthält, was besonders in der Anfangsphase nach der Implantation eine höhere Löslichkeit aufwies als das reine Kemmaterial aus alpha-TCP (EP 0237043 B1).

Übertroffen wurde deren chemische Löslichkeit durch ebenfalls bioaktive Werkstoffe auf der Basis von Calciumphosphaten, die zusätzlich Oxide des Kaliums, Natriums, Magnesiums und/oder Siliciums enthalten (EP 541546 B1) und glasig-kristallines Material sich auf die folgende Hauptkristallphasen gründen: Phase X, Rhenanit, Phase nach Ando (Phase A) bzw. von diesen zuvor genannten Phasen abgeleitete Mischkristalle.

Aus den genannten Schmelzprodukten, die zwar Orthophosphate und stets eine Glasphase enthalten und deren einzelne Phasenbestandteile homogen verteilt sind, wurden keine Zemente bekannt.

Anorganische Biozemente wurden vielmehr durch Pulvergemische aus verschiedenen Calciumphosphaten zusammengestellt, wie es z.B. bekannt ist aus EP 1153621 oder US 5129905.

Weiterhin ist aus der DE 19744809 C ein glasig-kristalliner Glaskörper mit Ca-K-Na-Phosphat-Hauptkristallphasen mit schneller Löslichkeit als Knochenersatz-werkstoff bekannt, hergestellt durch Zusammenschmelzen zweier Gläser, wobei eines der Gläser 20-55% CaO, 5-25 Na₂O, 0,01-20 K₂O, 0-15 MgO, 30-50 P₂O₅ und 0-15 SiO₂ enthält. Die Porosität wird durch Auslaugung der Borosilicatglasphase auf einen Rest von 0,05-2 Gew% erhalten. Die US-A-3922155 offenbart einen Prozeß zur Herstellung von biokompatiblen Glaskeramiken, die das Schmelzen einer Si0₂, Na₂O, K₂O, MgO, CaO und Ca₃(PO4)₂-Mischung sowie weitere Keramisierungsschritte umfaßt. Dabei werden für Knochen- und Zahnersatz günstige Apatitstrukturen ausgebildet.

Der Erfindung liegt die Aufgabe zugrunde, über ein Schmelzprodukt ein Pulver bereitzustellen, das mit reinem Wasser bzw. einer wässrigen Lösung direkt abbinden kann und vorzugsweise in der abgebundenen Form Erdalkali-Alkali-Orthophosphate vorweist, die eine höhere Löslichkeit aufweisen als apatitische Phasenbestandteile, insbesondere, wenn sie die Majorität eines Phasenbestandes ausmachen.

Erfindungsgemäß ist das Pulvergemisch für resorbierbare Calciumphosphat-Biozemente gekennzeichnet durch ein Fraktionsgemisch aus (bezogen auf das Gesamtvolumen des Pulvergemisches)
40-99 Vol-% Pulver mit 0,1 - 10µm Teilchengröße
1-20 Vol-% Pulver mit 10 - 43µm Teilchengröße
0-59 Vol-% Pulver mit 43 - 315µm Teilchengröße
wobei das Pulver aus aufgemahlenen, spontan kristallisierenden Schmelzen eines Materials mit kristallinen und röntgenamorphen Phasen besteht, das
a) nach ³¹P-NMR-Messungen Q₀-Gruppen von Orthophosphat und Q₁-Gruppen von Diphosphat enthält, wobei die Orthophosphate respektive Q₀-Gruppen, bezogen auf den Gesamtphosphorgehalt des Pulvergemisches, 65 bis 99,9 Gew-% betragen, und die Diphosphate respektive Q₁-Gruppen, bezogen auf den Gesamtphosphorgehalt des Pulvergemisches, 0,1 bis 35 Gew-% betragen, und
b) nach röntgendiffraktometrischen Messungen, bezogen auf das Gesamtgewicht des Pulvergemisches, 35 bis 99,9 Gew-% einer Hauptkristallphase, ausgewählt aus der Gruppe, bestehend aus Ca₂K₁₋ₓNa₁₊ₓ(PO₄)₂ , worin x=0,1 bis 0,9 ist, Ca₁₀Na(PO₄)₇, Ca₁₀K(PO₄)₇, Gemische davon und Mischkristalle im Umfang von Ca₁₀KₓNa₁₋ₓ(PO₄)₇ mit x=0 bis 1, enthält, und als Nebenkristallphase, bezogen auf das Gesamtgewicht des Pulvergemisches, 0,1 bis 20 Gew-% eines Stoffes enthält, ausgewählt aus der Gruppe, bestehend aus Na₂CaP₂O₇, K₂CaP₂O₇, Ca₂P₂O₇, NaPO₃, KPO₃ und Gemische davon, und
c) eine röntgenamorphe Phase enthält, die neben der Hauptkristallphase insgesamt 0,1 bis 65 Gew-% beträgt, bezogen auf das Gesamtgewicht des Pulvergemisches, und wobei das Pulver erhältlich ist, indem die für eine Gemengebildung geeigneten Substanzen 30 - 55 Gew-% CaO, 35 - 50 Gew-% P₂O₅, 1 - 20 Gew-% Na₂O, 0,5 - 20 Gew-% K₂O und 0,1 - 5 Gew-% MgO sowie ggf. bis zu 5 Gew-% SiO₂ vermengt werden, das Gemenge homogenisiert und getrocknet wird und einer stufenweisen Temperaturbehandlung von jeweils 1-2h bei 350-450 °C, 750-850°C und 950-1050°C unterworfen wird und das Gemisch bei 1550 bis 1650 °C geschmolzen, bei der Schmelztemperatur 10 bis 60 Minuten gehalten und schließlich spontan oder mit geregelter Temperatur abgekühlt und dann gemahlen wird.

Das Gemisch enthält bevorzugt 0,1-15 Gew-%, vorzugsweise 0,5-4 Gew-% Kettenphosphate, ausgewählt unter NaPO₃, KPO₃, Mischkristallen davon und Gemischen dieser, die bei ³¹P-NMR-Messungen Q₂-Gruppen anzeigen.

Der Anteil der Orthophosphate liegt vorzugsweise im Bereich von 40 bis 95 Gew-%, speziell bei 50 bis 90 Gew-%.

Der Anteil der Diphosphate liegt vorzugsweise im Bereich von 1 bis 22 Gew-%, speziell bei 5 bis 22 Gew-%.

Das Material (Pulvergemisch), das zur beschriebenen Zementbildung geeignet ist, besteht auf Basis von CaO, P₂O₅, Na₂O, K₂O, MgO und ggf. auch SiO₂ aus (in Gew-%): 30 bis 55 P₂O₅, 25 bis 50 CaO, 1 bis 20 Na₂O, 0,5 bis 20 K₂O, 0,1 bis 13 MgO, 0 bis 10 SiO₂.

Eine speziell bevorzugte Ausführungsform für eine Hauptkristallphase mit Ca₂K₁₋ₓNa₁₊ₓ(PO₄)₂ , worin x=0,1 bis 0,9 ist, enthält 40 bis 52 P₂O₅, 28 bis 33 CaO, 8,5 bis 13 Na₂O, 9,5 bis 15 K₂O, 1,5 bis 3 MgO, 0,1 bis 4 SiO_{2.}

Eine speziell bevorzugte Ausführungsform für eine Hauptkristallphase mit Ca₁₀KₓNa₁₋ₓ(PO₄)₇ mit x=0 bis 1 enthält 44 bis 54 P₂O₅, 34 bis 48 CaO, 1,5 bis 10,5 Na₂O, 1 bis 11 K₂O, 1,5 bis 3 MgO, 0,1 bis 4 SiO₂.

Nach einer bevorzugten Ausführungsform enthält das Pulvergemisch neben dem Pulver, das über eine Schmelze erzeugt wurde, zusätzlich bis 30 Gew-% eines Calciumphosphates, ausgewählt aus der Gruppe, bestehend aus alpha-Tricalciumphosphat, beta-Tricalciumphosphat und Gemische davon.

Nach einer weiteren bevorzugten Ausführungsform enthält das Pulvergemisch zusätzlich einen Wirkstoff, ausgewählt aus der Gruppe, bestehend aus Antibiotika, anderen pharmazeutischen Wirkstoffen, Desinfektionsmitteln, Bakteriostatika und Gemischen davon, vorzugsweise Antibiotika wie Tetracycline usw.

In Mischkristallen, die in dem erfindungsgemäßen Pulvergemisch enthalten sein können, kann das Element Ca durch Mg bis zu einem Anteil von 20 Gew-% ersetzt sein, bezogen auf das Gewicht des Pulvergemisches.

Eine besonders bevorzugte Ausführungsform der Erfindung besteht in einem zweikomponentigen Kit, in welchem das erfindungsgemäße Pulvergemisch eine Komponente und eine Wasserphase die andere Komponente darstellt.

Das erfindungsgemäße Pulvergemisch wird daher bevorzugt in eine wässrige Lösung, Suspension oder Paste überführt.

Der hier verwendete Begriff "röntgenamorphes Material" ist im allgemeinen nicht eindeutig definierbar. Unter röntgenamorph wird ein Material verstanden, dessen Struktur mit der üblichen XRD (Röntgendiffraktometrie) nicht mehr erfasst werden kann. Dabei kann es sich um sehr kleine geordnete Bereiche (mikrokristallin) als auch statistisch ungeordnete Bereiche handeln. Im Gegensatz zu XRD kann durch die ³¹P-NMR-Ergebnisse die Existenz jeder kristallinen Phase erfasst werden. Deshalb kann es bei der Mengenabschätzung zwischen NMR- und XRD-Ergebnissen zu gravierenden Unterschieden kommen. Besonders die Diphosphat- und Kettenphosphatanteile scheinen im vorliegenden Fall für dieses Phänomen symptomatisch zu sein; es werden in der Regel mit den ³¹P-NMR-Messungen deutlich höhere Anteile bestimmt als mit XRD bzw. mit XRD teilweise sogar keine Anteile. Dies zeigt eindrucksvoll, warum die ³¹P-NMR-Messungen für die Charakterisierung und letztlich Herstellung der erfindungsgemäßen Werkstoffe eine wesentliche Voraussetzung bilden.

Es können daher sowohl kristalline als auch röntgenamorphe Phasen innig vermischt vorliegen. Für die vorliegende Erfindung ist es ohne Belang, ob eine Phase neben der anderen vorliegt, oder ob eine Phase die andere umhüllt. Als "Hauptkristallphase" wird hier eine über Röntgendiffraktion ermittelte kristalline Phase bezeichnet, deren Mengenanteil wenigstens ein Drittel größer ist als der einer Nebenphase, wobei Konzentrationen von 20 und darunter, vorzugsweise unter 15 Gewichts-%, als Nebenkristallphasen bezeichnet werden und nicht überschritten werden sollten.

Zum besseren Verständnis muss noch darauf hingewiesen werden, dass zwar als Hauptkristallphase unter anderem "Ca₂KNa(PO₄)₂" identifiziert werden kann. Es gibt jedoch in den einzelnen Zusammensetzungen teilweise erhebliche Linienverschiebungen, die auf das wechselnde Verhältnis von Natrium zu Kalium bzw. auf den Einbau anderer lonen (wie Mg²⁺ oder SiO₄⁴⁻) zurückzuführen sind, so dass die Formel mit "Ca₂K₁₋ₓNa₁₊ₓ(PO₄)₂, wobei x=0,1 - 0,9 ist" einzusetzen ist.
Höhere Anteile Na sind bevorzugt, z.B. x=0,2 - 0,9.

Überraschenderweise wurde gefunden, dass Pulver aus dem erfindungsgemäßen Zusammensetzungsgebiet, wenn sie mit reinem Wasser bzw. wässrigen Lösungen vermischt werden, innerhalb von 2 bis 10 min abbinden können, ohne dass es weiterer Zusätze zwingend bedarf. Dieser Effekt kommt vermutlich dadurch zustande, indem die Pulver über hydraulische bzw. latent hydraulische Eigenschaften verfügen. Der Effekt tritt verstärkt auf, wenn neben den aufgeführten Hauptkristallphasen, vorzugsweise Ca₁₀Na(PO₄)₇, Ca₁₀K(PO₄)₇ bzw. Mischkristallphasen zwischen diesen Verbindungen, und röntgenamorphen Anteilen an Orthophosphaten auch Diphosphate als kristalline Diphosphate Na₂CaP₂O₇, K₂CaP₂O₇, Ca₂P₂O₇ bzw. röntgenamorphe Anteile im Schmelzprodukt vorliegen.
Ferner wurde überraschenderweise gefunden, dass sich diese Aussage durch ³¹P-NMR-Messungen eindeutig quantifizieren lasst.

Die ³¹P-NMR-Messungen, die mit einem supraleitenden Fourier NMR Spektrometer Avance DMX400 WB der Fa. Bruker BioSpin GmbH (Deutschland) durchgeführt wurden, zeigten, dass das Material aus 65 bis 99,99% Orthophosphat besteht, gebildet aus Calcium sowie ggf. Natrium, Kalium und Magnesium wobei dieser Orthophosphat-Anteil nachweislich durch ³¹P-NMR-Messungen (Q₀-Gruppen) erfolgt und sich auf kristallines und/oder röntgenamorphes Material in seiner Gesamtheit bezieht, sowie 0,1 bis 35% Diphosphat, gebildet aus Calcium sowie ggf. Natrium, Kalium und Magnesium, wobei dieser Diphosphat-Anteil nachweislich durch ³¹P-NMR-Messungen (Q₁-Gruppen) erfolgt und sich auf kristallines und/oder röntgenamorphes Material in seiner Gesamtheit bezieht, sowie 0 bis 10% Kettenphosphat, bestehend aus Natrium- und/oder Kaliumphosphat, wobei dieser Kettenphosphat-Anteil nachweislich durch ³¹P-NMR-Messungen (Q₂-Gruppen) erfolgt und sich insbesondere auf röntgenamorphes und ggf. kristallines Material in seiner Gesamtheit bezieht. Ferner kann in Abhängigkeit vom gewählten SiO₂-Zusatz 0 bis 10 % einer Silicatphase enthalten sein.

Die Diphosphatanteile resultieren aus einem vergleichsweise hohen Phosphatanteil im Vergleich zu den übrigen Bestandteilen. Dies könnte ursächlich dafür verantwortlich sein, dass die erfindungsgemäßen Zusammensetzungen im Vergleich zu bekannten resorbierbaren Werkstoffen sehr leicht einschmelzen und eine dünnflüssige Schmelze ergeben.

Obwohl es als Vorteil angesehen wird, dass der Abbindgang mit reinem Wasser erfolgen kann, ist der Zusatz von Kationen, insbesondere Natrium und Kalium, und Anionen, insbesondere Chloride, möglich.

Gegenstand der Erfindung ist auch die Verwendung des Pulvergemisches mit der o.g. Zusammensetzung zur Herstellung von resorbierbaren Calciumphosphat-Biozementen, wobei diese Biozemente nach dem Aushärten neben den jeweiligen Ausgangs-Hauptkristallphasen weniger als 20 Gew-% Hydroxylapatit (HA) und/oder präzipitierten Hydroxylapatit enthalten. Dies ist ein besonderer Vorteil, weil die Löslichkeit von HA geringer ist als die der erfindungsgemäßen Hauptphase. Ein bevorzugter HA-Anteil liegt unter 10 Gew-%.

Die Verwendung erfolgt im allgemeinen durch Vermischen eines Pulvergemisches mit o.g. Zusammensetzung mit reinem Wasser oder wässrigen Lösungen. Vorteilhaft kann der Abbindgang mit wässrigern Lösungen unter Zusatz von Kationen, insbesondere Natrium und Kalium, sowie Anionen, insbesondere Chloride erfolgen.

Der wässrigen Lösung können auch Kohäsionspromotoren und/oder Aushärtungsbeschleuniger zugesetzt werden, wobei als Kohäsionspromotoren Verbindungen aus den Gruppen der Hydroxyethylstärke, lösliche Stärke, Cyclodextrine, Alginate, Dextransulfate, Polyvinylpyrrolidon und/oder Hyaluronsäure ausgewählt werden können. Bevorzugt sind Stärke, Cyclodextrine und PVP.

Als Aushärtungsbeschleuniger wird der wässrigen Lösung bevorzugt Dinatriumhydrogenphosphat zugesetzt.

Das Mischungsverhältnis zwischen wässriger Lösung und Zementpulver liegt im Bereich von 0,15 bis 0,4 ml/g, vorzugsweise 0,18 bis 0,23 ml/g. So werden beispielsweise 0,5 ml Wasser auf 2,5 g Pulver eingesetzt, um ein Verhältnis von 0,2 ml/g zu erhalten.

Der zur Pulverbildung benötigte Werkstoff wird hergestellt, indem die für die Gemengebildung geeignete Substanzen
30 - 55 Gew-% CaO, 35 - 50 Gew-% P₂O₅, 1 - 20 Gew-% Na₂O, 0,5 - 20 Gew-% K₂O und 0,1 - 5 Gew-% MgO sowie ggf. bis zu 5 Gew-% SiO₂ vermengt werden, das Gemenge homogenisiert und getrocknet wird und einer stufenweisen Temperaturbehandlung von jeweils 1-2h bei 350-450 °C, 750-850°C und 950-1050°C unterworfen wird und das Gemisch bei 1550 bis 1650 °C geschmolzen, bei der Schmelztemperatur 10 bis 60 Minuten gehalten und schließlich spontan oder mit geregelter Temperatur abgekühlt und dann gemahlen wird.

Das Schmelzen erfolgt in einem geeigneten Tiegelmaterial, zum Beispiel bestehend aus einer Pt/Rh-Legierung. Die Schmelze wird, vorteilhaft nach einer Haltezeit von 10 bis 60 min, vergossen, und die erstarrte Schmelze wird je nach Verwendungszweck an der Luft (spontane Abkühlung) oder im Kühlofen (z.B. mit 1 bis 20 Grad/min) auf Raumtemperatur abgekühlt. Sie kann auch verblasen werden, wodurch kugelförmige Granulate direkt aus dem Schmelzfluss entstehen. Während des Abkühlens der Schmelzen erfolgt stets eine spontane Kristallisation. Als Gemengebestandteile können Oxide, Carbonate, Hydrogenphosphate und/oder Ortho-Phosphorsäure verwendet werden. Die ³¹P-NMR-Messungen zeigen dabei Unterschiede in den Spektren auf, die auf die verwendeten Rohstoffe Rückschlüsse zulassen bzw. respektive deren geringfügige Beimengungen an Eisen- bzw. Manganoxiden anzeigen.

Nach der Abkühlung wird der Werkstoff aufgemahlen und in Partikelfraktionen getrennt und vermischt zu
0,1 - 10µm im Volumenanteil von 40 - 99 Vol-%
10 - 40µm im Volumenanteil von 1 - 20 Vol-%
40 - 315µm Volumenanteil von 0 - 60 Vol-%,
wenn die Aufmahlung nicht dem gewünschten Spektrum entspricht.

Es können weitere Calciumphosphate zugesetzt werden, z.B. alpha-Tricalciumphosphat, beta-Tricalciumphosphat oder auch nicht bevorzugtes-Ca₅Na₂(PO₄)₄, CaNaPO₄ und Gemische davon. Diese werden in ähnlichen Kornfraktionen bis zu 30 Gew-% zugesetzt, was allerdings den Vorteil der innigen Vermischung der Phasen über den Schmelzzustand einschränken kann.

Vielmehr wird es als Vorteil betrachtet, dass die Schmelze aufgemahlen wird bzw. aus dieser einen Komponente ggf. ein Pulvergemisch hergestellt wird, und diese Komponente allein mit Wasser bzw., einer wässrigen Lösung, die ggf. Kohäsionspromotoren und/oder Aushärtungsbeschleuniger enthält, vermischt wird. Dabei erzielt man innerhalb von 2 bis 10 min ein Aushärten. Danach werden die in vitro erzeugten Probekörper sofort in SBF (simulated body fluid = simulierte Körperflüssigkeit) nach KOKUBO et al. [J.Biomed.Mater.Res. 24(1990)721-734] eingelagert und bleiben über einen Zeitraum von 4 Wochen in dieser Lösung mechanisch stabil und zerfallen nicht.

Dies ist auch der Fall, wenn dem Pulver bzw. Pulvergemisch vor dem Kontakt mit Wasser bzw. wässriger Lösung ggf. ein pharmazeutischer Wirkstoff, ein Antibiotikum, ein Desinfektionsmittel oder Bakteriostatikum zugesetzt wurde.

Die Erfindung wird nachstehend durch Beispiele näher erläutert. Alle Prozentangaben sind auf das Gewicht bezogen, sofern nicht anderes angegeben ist.

In der dazugehörigen Zeichnung zeigen
Fig. 1: XRD-Spektren des erfindungsgemäßen Materials 40-30-30 mit der Zusammensetzung gemäß Beispiel 4 und den Phasen gemäß Beispiel 6
Fig. 2: XRD-Spektren des erfindungsgemäßen Materials 50-25-25 mit der Zusammensetzung gemäß Beispiel 4 und den Phasen gemäß Beispiel 6.
Fig. 3: XRD-Spektren der Zusammensetzung mit Code GA1 als Ausgangsmaterial (1) und nach 4-wöchiger Lagerung in SBF (2)
Fig. 4a: Vergleich der ³¹P-MAS-NMR-Spektren der Probe 40-30-30 vor und nach der Lagerung in SBF gemäß Beispiel 12
Fig. 4b: Vergleich der ³¹P-MAS-NMR-Spektren der Probe 40-30-30 vor und nach der Lagerung in SBF mit vergrößertem Ausschnitt -16ppm bis -30ppm

### Beispiel 1

Es wurden folgende Materialien synthetisiert nach den Vorgaben in Gew-%:

| Code | CaO | MgO | P₂O₅ | Na₂O | K₂O | SiO₂ |
|---|---|---|---|---|---|---|
| GA 1 | 30,67 | 2,45 | 43,14 | 9,42 | 14,32 | 0,00 |
| GA 2 | 29,92 | 2,39 | 44,53 | 9,19 | 13,97 | 0,00 |
| GA 3 | 29,21 | 2,33 | 45,85 | 8,97 | 13,64 | 0,00 |

Zum besseren Verständnis kann man diese Schmelzabfolge auch so darstellen:
Ga 1; Ga 2(=GA 1 + 2,5% P₂O₅); GA 3(=GA 1 + 5% P₂O₅)°

Die Gemenge wurden wie folgt eingewogen:

| Code | CaCO₃ In g | MgO in g | 85%ige-H₃PO₄ in ml | Na₂CO₃ in g | K₂CO₃ in g | SiO₂ in g |
|---|---|---|---|---|---|---|
| GA 1 | 54,74 | 2,45 | 41,48 | 16,11 | 21,01 | 0 |
| GA 2 | 53,40 | 2,39 | 42,82 | 15,72 | 20,50 | 0 |
| GA 3 | 52,13 | 2,33 | 44,09 | 15,34 | 20,01 | 0 |

Zunächst werden die Komponenten an Calcium, Magnesium, Natrium und Kalium, ggf. auch Silicium eingewogen. Nach dem Einwiegen wird das jeweilige Gemenge in einem Taumelmischer eine Stunde lang gemischt. Danach wird das Gemenge mit der 85%igen Ortho-Phosphorsäure versetzt, gut gemörsert und gerührt sowie eine Stunden lang bei 100°C getrocknet, erneut gemörsert und wiederum eine Stunde bei 100°C im Trockenschrank aufbewahrt. Sodann wurde das Gemenge erneut gemörsert und in einen Pt/Rh-Schale gefüllt und auf 400°C erhitzt, nach Erreichen dieser Temperatur eine Stunde lang bei dieser Temperatur gehalten, sodann auf 800°C hochgeheizt, nach Erreichen dieser Temperatur wiederum eine Stunde lang bei dieser Temperatur gehalten und sodann auf 1000°C erhitzt und nach Erreichen dieser Temperatur eine Stunde lang bei dieser Temperatur gehalten. Dieser Sinterkuchen wurde an Luft abgekühlt und erneut zum Zwecke der Homogenisierung gemörsert. Dieses vorbehandelte Gemenge wurde sodann in einen Platin-Tiegel gefüllt und im Schmelzofen auf 1600°C erhitzt. Nach Erreichen dieser Temperatur wurde die Schmelze eine halbe Stunde lang bei dieser Temperatur belassen. Die dünnflüssigen homogenen Schmelzen wurden dann auf eine Stahlplatte gegossen und mit einer weiteren Stahlplatte zu einer salzartig erstarrten Platte verpresst. Die dabei erfolgende Kristallisation verleiht den Schmelzkörpern eine opake, weiße Farbe.

### Beispiel 2

Nach dem gleichen Herstellungsprozedere, wie in Beispiel 1 beschrieben, d.h. Gemengeerzeugung mit Calciumcarbonat, Natriumcarbonat, Kaliumcarbonat und Ortho-Phosphorsäure wurden folgende Zusammensetzungen nach den folgenden Vorgaben in Gew-%:

| Code | CaO | MgO | P₂O₅ | Na₂O | K₂O | SiO₂ |
|---|---|---|---|---|---|---|
| GA 4 | 31,54 | 1,19 | 42,37 | 9,17 | 13,95 | 1,78 |
| GA 5 | 30,79 | 1,16 | 43,74 | 8,95 | 13,62 | 1,73 |

Es ergaben sich für alle Zusammensetzungskompositionen dünnflüssige Schmelzen, die spontan beim Abkühlen kristallisierten. Die Kristallisationsprodukte wiesen eine weiße Farbe auf.

### Beispiel 3

Eine weitere Herstellungsmöglichkeit besteht u.a. darin, dass die gesamte Phosphor- bzw. Phosphatmenge oder wie im vorliegenden Beispiel ein Teil durch einen Calciumträger eingebracht werden kann. Es wurde folgende Zusammensetzung synthetisiert nach den Vorgaben in Gew-%:

| Code | CaO | MgO | P₂O₅ | Na₂O | K₂O | SiO₂ |
|---|---|---|---|---|---|---|
| GA 1 | 30,67 | 2,45 | 43,14 | 9,42 | 14,32 | 0,00 |

Das Gemenge wurde wie folgt eingewogen:

| Code | CaCO₃ in g | Magnesiumhydroxidcarbonat in g | 85%ige-H₃PO₄ in ml | Na₂CO₃ in g | K₂CO₃ in g | CaHPO₄ in g |
|---|---|---|---|---|---|---|
| GA 1 | 0,00 | 5,13 | 4,25 | 16,11 | 21,00 | 74,43 |

Das Gemenge wurde nach dieser Vorgabe eingewogen, eine Stunde lang im Taumelmischer gemischt, mit der Phosphorsäure versetzt, eine Stunde bei 100°C getrocknet, an der Luft abgekühlt und gemörsert. Diese Gemenge wurde in einen Platin-Tiegel gefüllt und in einen auf 450°C vorgeheizten Ofen gestellt und 16 Stunden lang bei dieser Temperatur gehalten. Der Tiegel wurde entnommen und der Ofen nunmehr auf 750°C vorgeheizt, 4 Std. gehalten und in den auf 950°C vorgeheizten Ofen gebracht und 6 Stunden lang gehalten. Sodann wurde die Probe auf 1600°C hochgeheizt und eine halbe Stunde nach Erreichen dieser Temperatur bei dieser Temperatur gehalten. Die dünnflüssige homogene Schmelze wurde dann auf eine Stahlplatte gegossen und mit einer weiteren Stahlplatte zu einer salzartig erstarrten Platte verpresst. Die dabei erfolgende Kristallisation verleiht den Schmelzkörpern eine opake, weiße Farbe. In Abhängigkeit von der eingesetzten CaHPO₄₋Komponente und deren Verunreinigung an Eisen und/oder Mangan, kann eine Verfärbung beobachtet werden.

Es ist auch möglich die Schmelze nach dem Schmelzvorgang (1600°C, 0,5h Halten) in einem Wasserbad abzuschrecken (Fritten), um das weitere Zerkleinern des Schmelzproduktes zu erleichtern.

### Beispiel 4

Nach dem gleichen Herstellungsprozedere, wie in Beispiel 1 beschrieben, d.h. Gemengeerzeugung mit Calciumcarbonat, Natriumcarbonat, Kaliumcarbonat und Ortho-Phosphorsäure wurden folgende Zusammensetzungen nach den folgenden Vorgaben in Gew-%:

| Code | CaO | MgO | P₂0₅ | Na₂0 | K₂O | SiO₂ |
|---|---|---|---|---|---|---|
| 50-25-25 | 39,86 | 1,25 | 46,82 | 4,79 | 7,28 | 0 |
| 60-20-20 | 37,99 | 1,49 | 46,08 | 5,73 | 8,71 | 0 |
| 40-30-30 | 41,74 | 1 | 47,58 | 3,84 | 5,84 | 0 |
| 80-10-10 | 34,31 | 1,97 | 44,6 | 7,59 | 11,53 | 0 |
| 60-30-10 | 39,05 | 1,48 | 45,13 | 5,69 | 8,65 | 0 |
| 50-40-10 | 41,43 | 1,23 | 45,39 | 4,74 | 7,21 | 0 |
| 50-32,5-17,5 | 40,65 | 1,24 | 46,1 | 4,77 | 7,24 | 0 |
| 40-50-10 | 43,8 | 0,99 | 45,65 | 3,79 | 5,77 | 0 |
| 40-40-20 | 42,78 | 0,99 | 46,61 | 3,82 | 5,8 | 0 |
| 30-50-20 | 45,16 | 0,75 | 46,88 | 2,86 | 4,35 | 0 |
| 20-50-30 | 46,55 | 0,5 | 48,11 | 1,92 | 2,92 | 0 |
| 30-0-70 | 40,1 | 0,73 | 52,04 | 2,83 | 4,3 | 0 |
| 50-0-50 | 37,4 | 1,23 | 49,5 | 4,71 | 7,16 | 0 |
| 70-0-30 | 34,71 | 1,72 | 46,96 | 6,59 | 10,02 | 0 |
| 50-40-10-Si | 41,4 | 1,3 | 44,4 | 10,2 | 1,7 | 1 |

Es ergaben sich für alle Zusammensetzungskompositionen dünnflüssige Schmelzen, die spontan beim Abkühlen kristallisierten. Die Kristallisationsprodukte wiesen eine weiße Farbe auf.

### Beispiel 5

Von den Proben nach Beispiel 1 und nach Beispiel 2 wurden ³¹P-MAS-NMR-Spektren mit einer Wartezeit von 120s zwischen den Einzelpulsen aufgenommen. Die Probenrotationsgeschwindigkeit betrug 12,5 kHz.
Die quantitative Zusammensetzung der Proben in Bezug auf den Phosphatanteil ist in folgender Tabelle angegeben:

| Code | Orthophosphat-Anteil [(PO₄)³⁻] in % | Diphosphat-Anteil [(P₂O₇)²⁻] in % | Kettenphosphat-Anteil [vorrangig (PO₃)¹⁻] in % |
|---|---|---|---|
| GA 1 | 99,5-96 | 0,5-4 | - |
| GA 2 | 88 | 12 | - |
| GA 3 | 79 | 21 | - |
| | | | |
| GA 4 | 95 | 5 | - |
| GA 5 | 89 | 11 | - |

Während von den angegebenen Proben jeweils nur eine Probe untersucht wurde, bezieht sich das Ergebnis mit der angegebenen Bandbreite für die Zusammensetzung GA 1 auf insgesamt drei Chargen, wobei eine Charge nach der unter Beispiel 3 genannten Herstellungsmethode synthetisiert wurde.

### Beispiel 6

Von ausgewählten Proben nach Beispiel 4 wurden ³¹P-MAS-NMR-Spektren mit einer Wartezeit von 120s zwischen den Einzelpulsen aufgenommen. Die Probenrotationsgeschwindigkeit betrug 12,5 kHz.
Die quantitative Zusammensetzung der Proben bezogen auf Phosphor ist in folgender Tabelle angegeben:

| Code | Orthophosphat-Anteil [(PO₄)³⁻] in % | Diphosphat-Anteil [(P₂O₇)²⁻] in % | Kettenphosphat-Anteil [vorrangig (PO₃)¹⁻] in % |
|---|---|---|---|
| 50-40-10 | 92,5 | 7,5 | - |
| 50-32,5-17 | 93 | 7 | - |
| 50-25-25 | 86 | 14 | - |
| 40-50-10 | 91 | 9 | - |
| 40-40-20 | 84 | 16 | - |
| 40-30-30 | 82,5 | 13 | 4,5 |
| 30-50-20 | 88 | 9 | 3 |
| 20-50-30 | 73 | 27 | - |
| 60-20-20 | 92 | 8 | - |

### Beispiel 7

In einer Planetenmühle (200 U/min) im Zirkonoxidbecher (250ml) wurde unter den Bedingungen drei Mal 20min lang 30g des geschmolzenen Produktes der Zusammensetzung mit Code-Bezeichnung GA 1 nach Beispiel 1 aufgemahlen.

Das Resultat ist in der folgenden Tabelle zu dargestellt.

| Code | D₅₀-Wert [in µm] |
|---|---|
| GA 1 | 8,79 |

### Beispiel 8

In einer Planetenmühle (200 U/min) im Zirkonoxidbecher (250ml) werden unter jeweils den gleichen Bedingungen (drei Mal 20min lang) je 30g der geschmolzenen Produkte der Zusammensetzungen mit der Code-Bezeichnung 30-50-20, 40-30-30, 50-25-25 und 60-20-20 nach Beispiel 4 aufgemahlen:

| Code | D₅₀-Wert [in µm] |
|---|---|
| 30-50-20 | 4,21 |
| 40-30-30 | 9,08 |
| 50-25-25 | 8,78 |
| 60-20-20 | 8,30 |

### Beispiel 9

Es wurden jeweils 2,5g Pulver der Zusammensetzungen mit dem Code 50-25-25 oder 40-30-30 oder 60-20-20 hergestellt nach Beispiel 8, mit 0,5ml Wasser (E-pur-Wasser) vermischt und in Formen der Abmessungen 10 mm Durchmesser und 8 mm Höhe eingebracht. Nach drei Minuten war das Pulver/Wasser-Gemisch so abgebunden, dass es komplikationslos entformt werden konnte. Nach insgesamt 5 Minuten nach dem Anrühren wurden die Proben in SBF-Lösung eingelagert (pH=7,4; 37°C im Inkubator bei 75 Umin⁻¹), ohne dass sie dabei zerfielen. Die Lagerung in SBF erfolgte dabei wahlweise und abhängig von den weiteren Eigenschaftsuntersuchungen 1 Tag oder 4 Wochen.

### Beispiel 10

Es wurden jeweils 2g Pulver der Zusammensetzung mit dem Code GA 1 hergestellt nach Beispiel 7, und 0,2g Ca(H₂PO₄)₂•H₂O mit 0,5ml Wasser (E-pur-Wasser) vermischt und in Formen der Abmessungen 10 mm Durchmesser und 8 mm Höhe eingebracht. Nach drei Minuten war das Pulver/Wasser-Gemisch so abgebunden, dass es komplikationslos entformt werden konnte. Nach insgesamt 5 Minuten nach dem Anrühren wurden die Proben in SBF-Lösung eingelagert (pH=7,4; 37°C im Inkubator bei 75 Umin⁻¹), ohne dass sie dabei zerfielen. Die Lagerung in SBF erfolgte dabei wahlweise und abhängig von den weiteren Eigenschaftsuntersuchungen 1 Tag oder 4 Wochen.

### Beispiel 11

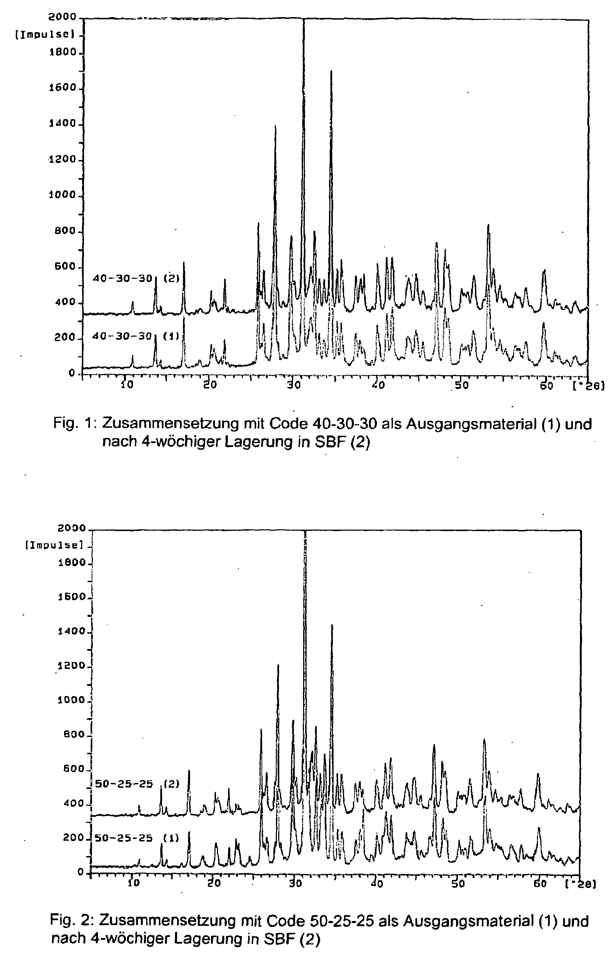

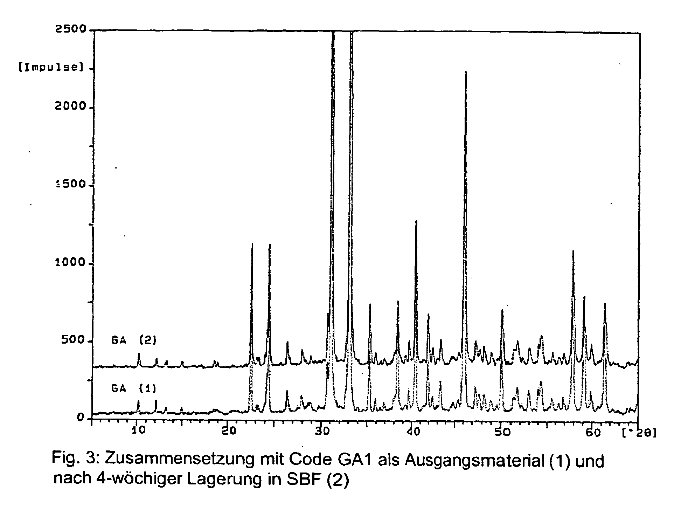

Von Proben nach Beispiel 8 und 9 (Zusammensetzung mit Code-Bezeichnung 40-30-30 und 50-25-25; Fig. 1 und Fig. 2) und Beispiel 7 und 10 (GA 1; Fig. 3) wurden Röntgendiffraktionsuntersuchungen (XRD) durchgeführt, d.h. vom Ausgangspulver und von erneut pulverisierten Proben, die 4 Wochen lang in SBF eingelagert wurde. Die Ergebnisse sind in den Fig. 1 bis 3 dargestellt und zeigen, dass sich die XRD Spektren nicht wesentlich unterscheiden und dass insbesondere kein HA, PHA oder Ca-d-HA in den Proben vorliegt.

### Beispiel 12

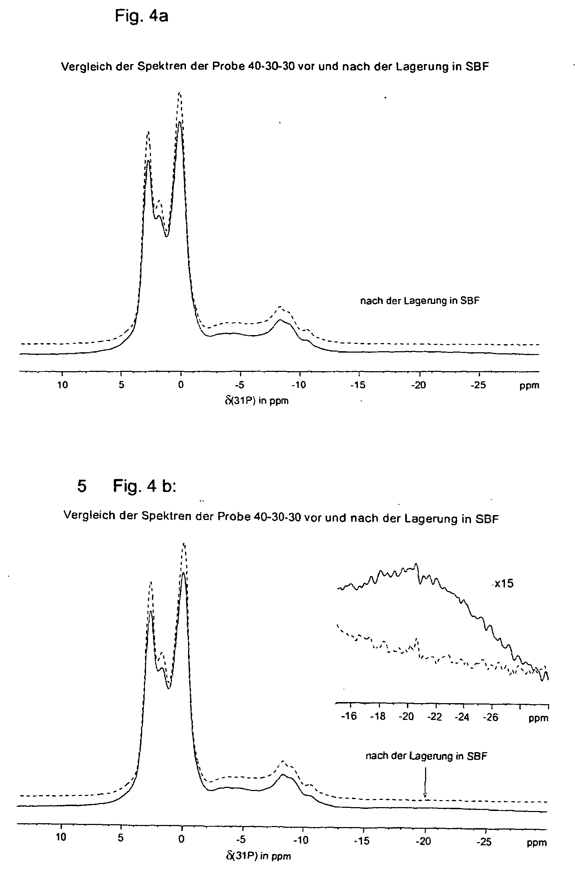

Material der Code-Bezeichnung 50-25-25 und 40-30-30 (Fig. 4a - b) nach Beispiel 8 und 11 wurde als Ausgangsmaterial und nach 4-wöchiger SBF-Einlagerung mit Hilfe der ³¹P-MAS-NMR untersucht. Das Ergebnis zeigt, dass zwischen dem Ausgangsmaterial und der 4 Wochen lang in SBF gelagerten Probe im wesentlichen keine Unterschiede bestehen und lediglich die Kettenphosphatanteile in der 15-fachen Verstärkung (vergleiche Insert in Fig. 4 b) nicht mehr vorhanden sind.

### Beispiel 13

Von Proben des Materials der Code-Bezeichnung 40-30-30 nach Beispiel 11 wurde nach 1-tägiger SBF-Einlagerung sofort im feuchten Zustand die Druckfestigkeit zu 3,7 MPa und nach weiterer 1-tägiger Trocknung bei 110 °C zu 6,5 MPa bestimmt.

### Beispiel 14

Von Proben des Materials der Code-Bezeichnung 40-30-30 nach Beispiel 11 wurde nach 4-wöchiger SBF-Einlagerung und weiterer 1-tägiger Trocknung bei 110 °C die Druckfestigkeit zu 6,4 MPa bestimmt. Von Proben des Materials der Code-Bezeichnung 50-25-25 nach Beispiel 11 wurde nach 4-wöchiger SBF-Einlagerung und weiterer 1-tägiger Trocknung bei 110 °C die Druckfestigkeit zu 6,4 MPa bestimmt.

## Patentansprüche

1. Pulvergemisch für resorbierbare Calciumphosphat-Biozemente, **gekennzeichnet durch** ein Fraktionsgemisch aus (bezogen auf das Gesamtvolumen des Pulvergemisches)
40-99 Vol-% Pulver mit 0,1 - 10µm Teilchengröße
1-20 Vol-% Pulver mit 10 - 43µm Teilchengröße
0-59 Vol-% Pulver mit 43 - 315µm Teilchengröße
wobei das Pulver aus aufgemahlenen, spontan kristallisierenden Schmelzen eines Materials mit kristallinen und röntgenamorphen Phasen besteht, das
a) nach ³¹P-NMR-Messungen Q₀-Gruppen von Orthophosphat und Q₁-Gruppen von Diphosphat enthält, wobei die Orthophosphate respektive Q₀-Gruppen, bezogen auf den Gesamtphosphorgehalt des Pulvergemisches, 65 bis 99,9 Gew-% betragen, und die Diphosphate respektive Q₁-Gruppen, bezogen auf den Gesamtphosphorgehalt des Pulvergemisches, 0,1 bis 35 Gew-% betragen, und
b) nach röntgendiffraktometrischen Messungen, bezogen auf das Gesamtgewicht des Pulvergemisches, 35 bis 99,9 Gew-% einer Hauptkristallphase, ausgewählt aus der Gruppe, bestehend aus Ca₂K₁₋ₓNa₁₊ₓ(PO₄)₂, worin x=0,1 bis 0,9 ist, Ca₁₀Na(PO₄)₇, Ca₁₀K(PO₄)₇, Gemische davon und Mischkristalle im Umfang von Ca₁₀KₓNa₁₋ₓ(PO₄)₇ mit x=0 bis 1, enthält, und als Nebenkristallphase, bezogen auf das Gesamtgewicht des Pulvergemisches, 0,1 bis 20 Gew-% eines Stoffes enthält, ausgewählt aus der Gruppe, bestehend aus NaPO₃, KPO₃ und Gemische davon, und
c) eine röntgenamorphe Phase enthält, die neben der Hauptkristallphase insgesamt 0,1 bis 65 Gew-% beträgt, bezogen auf das Gesamtgewicht des Pulvergemisches und wobei das Pulver erhältlich ist, indem die für eine Gemengebildung geeigneten Substanzen 30 - 55 Gew-% CaO, 35 - 50 Gew-% P₂O₅, 1 - 20 Gew-% Na₂O, 0,5 - 20 Gew-% K₂O und 0,1 - 5 Gew-% MgO sowie ggf. bis zu 5 Gew-% SiO₂ vermengt werden, das Gemenge homogenisiert und getrocknet wird und einer stufenweisen Temperaturbehandlung von jeweils 1-2h bei 350-450 °C, 750-850°C und 950-1050°C unterworfen wird und das Gemisch bei 1550 bis 1650 °C geschmolzen, bei der Schmelztemperatur 10 bis 60 Minuten gehalten und schließlich spontan oder mit geregelter Temperatur abgekühlt und dann gemahlen wird.

2. Pulvergemisch nach Anspruch 1, **dadurch gekennzeichnet, dass** das Gemisch 0,1 - 15 Gew-%, vorzugsweise 0,5 - 4 Gew-% Kettenphosphate enthält, ausgewählt unter NaPO₃, KPO₃, Mischkristallen davon und Gemischen dieser, die bei ³¹P-NMR-Messungen Q₂-Gruppen anzeigen.

3. Pulvergemisch nach Anspruch 1, **dadurch gekennzeichnet, dass** der Anteil der Orthophosphate im Bereich von 40 bis 95 Gew-% liegt, vorzugsweise 50 bis 90 Gew-%.

4. Pulvergemisch nach Anspruch 1, **dadurch gekennzeichnet, dass** der Anteil der Diphosphate im Bereich von 1 bis 22 Gew-% liegt, vorzugsweise im Bereich von 5 bis 22 Gew-%.

5. Pulvergemisch nach Anspruch 1, **dadurch gekennzeichnet, dass** es im geschmolzenem oder aufgemahlenem Zustand besteht aus in Gew-%:
| | | | |
|---|---|---|---|
| 30 | bis | 55 | P₂O₅ |
| 25 | bis | 50 | CaO |
| 1 | bis | 20 | Na₂O |
| 0,5 | bis | 20 | K₂O |
| 0,1 | bis | 13 | MgO |
| 0,0 | bis | 10 | SiO₂ |
wobei MgO oder SiO₂ oder ein Gemisch davon wenigstens 1 Gew-% beträgt, und den entsprechenden Kristallphasen.

6. Pulvergemisch nach Anspruch 1, **dadurch gekennzeichnet, dass** es bis zu 10% einer silicatischen Phase enthält.

7. Pulvergemisch nach einem der Ansprüche 1 bis 3, **dadurch gekennzeichnet, dass** es neben dem Pulver, das über eine Schmelze erzeugt wurde, zusätzlich bis 30 Gew-% alpha-Tricalciumphosphat, beta-Tricalciumphosphat oder Gemische davon enthält.

8. Pulvergemisch nach Anspruch 1 bis 7, **dadurch gekennzeichnet, dass** es zusätzlich einen Wirkstoff enthält, ausgewählt aus der Gruppe, bestehend aus Antibiotika, anderen pharmazeutischen Wirkstoffen, Desinfektionsmitteln, Bakteriostatika und Gemischen davon.

9. Pulvergemisch nach Anspruch 1 bis 8, **dadurch gekennzeichnet, dass** es in Form einer wässrigen Lösung, Suspension oder Paste vorliegt.

10. Pulvergemisch nach Anspruch 1, **dadurch gekennzeichnet, dass** in Mischkristallen das Element Ca durch Mg bis zu einem Anteil von 20 Gew-% ersetzt ist, bezogen auf das Gewicht des Pulvergemisches.

11. Pulvergemisch nach Anspruch 1 bis 10, **dadurch gekennzeichnet, dass** es in einem zweikomponentigen Kit vorliegt, wobei das Pulver eine Komponente und eine Wasserphase die andere Komponente darstellt.

12. Verwendung des Pulvergemisches nach einem der Ansprüche 1-11 zur Herstellung von resorbierbaren Calciumphosphat-Biozementen, enthaltend nach dem Aushärten des Zements neben den jeweiligen Ausgangs-Hauptkristallphasen weniger als 20 Gew-% Hydroxylapatif und/oder präzipitierten Hydroxylapatit.

13. Verwendung nach Anspruch 12 durch Vermischen eines Pulvergemisches nach Anspruch 1 mit reinem Wasser oder wässrigen Lösungen.

14. Verwendung nach Anspruch 13, **dadurch gekennzeichnet, dass** der Abbindgang mit wässrigem Lösungen unter Zusatz von Kationen, insbesondere Natrium und Kalium, sowie Anionen, insbesondere Chloride erfolgt.

15. Verwendung nach Anspruch 14, **dadurch gekennzeichnet, dass** der wässrigen Lösung Kohäsionspromotoren und/oder Aushärtungsbeschleuniger zugesetzt werden.

16. Verwendung nach Anspruch 15, **dadurch gekennzeichnet, dass** als Kohäsionspromotoren Verbindungen aus den Gruppen der Hydroxyethylstärke, lösliche Stärke, Cyclodextrine, Alginate, Dextransulfate, Polyvinylpyrrolidon und/oder Hyaluronsäure ausgewählt werden, und dass als Aushärtungsbeschleuniger der wässrigen Lösung Dinatriumhydrogenphosphat zugesetzt wird.

17. Verwendung nach Anspruch 13, **dadurch gekennzeichnet, dass** das Mischungsverhältnis zwischen wäßriger Lösung und Zementpulver im Bereich von 0,15 bis 0,4 ml/g liegt, vorzugsweise 0,18 bis 0,23 ml/g.

18. Verwendung nach Anspruch 12 einer Mischung in Form einer wässrigen Lösung, Suspension oder Paste zur Herstellung von biologisch abbaubarem Knochenersatzmaterial, das sowohl in vivo zur Defektfüllung eingesetzt werden kann als auch in vitro zur Zellzüchtung beim Tissue Engineering.

19. Biologisch abbaubares Implantat mit offener oder geschlossener Porosität, hergestellt aus einer ex vivo ausgehärteten Mischung in Form einer wässrigen Lösung, Suspension oder Paste nach Anspruch 1.

## Claims

1. A powder mixture for resorbable calcium phosphate biocements, **characterized by** a fraction mixture consisting of (relative to the total volume of the powder mixture):
40-99% by volume of powder having a particle size of 0.1-10µm
1-20% by volume of powder having a particle size of 10-43µm
0-59% by volume of powder having a particle size of 43-315µm
which powder is obtained by grinding the spontaneously crystallizing melts of a material comprising crystalline and X-ray amorphous phases, which material
a) according to ³¹P-NMR measurements, contains Q₀-groups of orthophosphate and Q₁-groups of diphosphate, the orthophosphates or Q₀-groups making up 65 to 99.9% by weight relative to the total phosphorus content of the powder mixture and the diphosphates or Q₁-groups making up 0.1 to 35% by weight relative to the total phosphorus content of the powder mixture, and
b) according to X-ray diffractometric measurements and relative to the total weight of the powder mixture, contains 35 to 99.9% by weight of a main crystal phase selected from the group consisting of Ca₂K₁₋ₓNa₁₊ₓ(PO₄)₂, where x = 0.1 to 0.9, Ca₁₀Na(PO₄)₇, Ca₁₀K(PO₄)₇, mixtures thereof and mixed crystals according to the general formula Ca₁₀KₓNa₁₋ₓ(PO₄)₇, where x = 0 to 1, and 0.1 to 20% by weight of a substance selected from the group consisting of Na₂CaP₂O₇, K₂CaP₂O₇, Ca₂P₂O₇, NaPO₃, KPO₃ and mixtures thereof as a secondary crystal phase, and
c) besides the main crystal phase, contains an X-ray amorphous phase which in total makes up 0.1 to 65% by weight relative to the total weight of the powder mixture and wherein the powder is obtainable by combining the substances suitable for preparing the mixture to be melted, i.e. 30-55% by weight CaO, 35-50% by weight P₂O₅, 1-20% by weight Na₂O, 0.5-20% by weight K₂O and 0.1-5% by weight MgO and optionally up to 5% by weight SiO₂, homogenizing and drying the mixture and subjecting it to a step-by-step thermal treatment lasting 1-2h at 350-450°C, 750-850°C and 950-1,050°C respectively, melting the mixture at between 1,550 and 1,650°C, holding it at the melting temperature for between 10 and 60 minutes and finally cooling the mixture in a spontaneous or temperature-controlled manner and grinding it.

2. A powder mixture according to Claim 1, wherein said mixture contains 0.1-15% by weight, preferably 0.5-4% by weight chain phosphates selected from among NaPO₃, KPO₃, mixed crystals thereof and mixtures of the foregoing, which are indicated by Q₂-groups in ³¹P-NMR measurements.

3. A powder mixture according to Claim 1, wherein the orthophosphates make up 40 to 95% by weight, preferably 50 to 90% by weight.

4. A powder mixture according to Claim 1, wherein the diphosphates make up 1 to 22% by weight, preferably 5 to 22% by weight.

5. A powder mixture according to Claim 1, wherein in the melted or ground state said mixture consists of (in % by weight):
| | | | |
|---|---|---|---|
| 30 | to | 55 | P₂O₅ |
| 25 | to | 50 | CaO |
| 1 | to | 20 | Na₂O |
| 0.5 | to | 20 | K₂O |
| 0.1 | to | 13 | MgO |
| 0.0 | to | 10 | SiO₂ |
MgO or SiO₂ or a mixture thereof making up at least 1% by weight, and the corresponding crystal phases.

6. A powder mixture according to Claim 1, wherein said mixture contains up to 10% of a silicate phase.

7. A powder mixture according to any of Claims 1 through 3, wherein said mixture additionally contains up to 30% by weight alpha-tricalcium phosphate, beta-tricalcium phosphate and/or mixtures thereof besides the powder obtained by a melting process.

8. A powder mixture according to Claims 1 through 7, wherein said mixture additionally contains an active agent selected from the group consisting of antibiotics, other pharmaceutical active agents, disinfectants, bacteriostats and mixtures thereof.

9. A powder mixture according to Claims 1 through 8, wherein said mixture is provided in the form of an aqueous solution, a suspension or a paste.

10. A powder mixture according to Claim 1, wherein in mixed crystals the element Ca is replaced by Mg in an amount ranging up to 20% by weight relative to the weight of the powder mixture.

11. A powder mixture according to Claims 1 through 11, wherein said mixture is provided in a two-component kit wherein one component is said powder and the other component is made up of a water phase.

12. The use of the powder mixture according to any of Claims 1 through 11 for manufacturing resorbable calcium phosphate biocements which contain less than 20% by weight hydroxyapatite (HA) and/or precipitated hydroxyapatite besides their initial main crystal phases once the setting process of the cement is finished.

13. The use according to Claim 12 by mixing a powder mixture according to Claim 1 with pure water or aqueous solutions.

14. The use according to Claim 13, wherein the setting process takes place using aqueous solutions additionally containing cations, particularly sodium and potassium, and anions, particularly chlorides.

15. The use according to Claim 14, wherein cohesion promoters and/or setting accelerators are added to the aqueous solution.

16. The use according to Claim 15, wherein compounds from the groups of hydroxyethyl starch, soluble starch, cyclodextrins, alginates, dextran sulphates, polyvinylpyrrolidone and/or hyaluronic acid are selected as cohesion promoters and disodium hydrogen phosphate is added to the aqueous solution as a setting accelerator.

17. The use according to Claim 14, wherein the mixture relation between the aqueous solution and the cement powder is ranging between 0.15 and 0.4ml/g, preferably 0.18 and 0.23ml/g.

18. The use according to Claim 10 of a mixture provided in the form of an aqueous solution, a suspension or a paste for manufacturing a biodegradable bone replacement material which can be used both for filling defects in vivo and for cultivating cells in vitro in tissue engineering.

19. A biodegradable implant having an open-pore or closed-pore structure, which implant is manufactured using a mixture according to Claim 1 which is provided in the form of an aqueous solution, a suspension or a paste and has set ex vivo.

## Revendications

1. Mélange pulvérulent pour biociments résorbables au phosphate de calcium, **caractérisé par** un mélange de fractions comprenant (par rapport au volume total de mélange pulvérulent)
40-99% en volume de poudre avec une taille de particules 0,1 - 10µm
1-20% en volume de poudre avec une taille de particules 10 - 43µm
0-59% en volume de poudre avec une taille de particules 43 - 315µm
sachant que la poudre est constituée de masses fondues à cristallisation spontanée broyées d'un matériau à phases cristallines et amorphes aux rayons X qui,
a) selon des mesures de RMN ³¹P, contient des groupes Q₀ d'orthophosphate et des groupes Q₁ de diphosphate sachant que les orthophosphates, respectivement les groupes Q₀, représentent de 65 à 99,9% en poids par rapport à la teneur totale en phosphore du mélange pulvérulent, et que les diphosphates, respectivement les groupes Q₁, représentent de 0,1 à 35% en poids par rapport à la teneur totale en phosphore du mélange pulvérulent, et
b) selon des mesures de diffraction aux rayons X, contient, par rapport à la masse totale de mélange pulvérulent, 35 à 99,9% en poids d'une phase cristalline principale choisie dans le groupe comprenant Ca₂K₁₋ₓNa₁₊ₓ(PO₄)₂, où x=0,1 à 0,9, Ca₁₀Na(PO₄)₇, Ca₁₀K(PO₄)₇, des mélanges de ceux-ci et des cristaux mixtes à la périphérie de Ca₁₀KₓNa₁₋ₓ(PO₄)₇ avec x=0 à 1, et qui contient comme phase cristalline secondaire, par rapport à la masse totale de mélange pulvérulent, de 0,1 à 20 % en poids d'une substance choisie dans le groupe comprenant NaPO₃, KPO₃ et des mélanges de ceux-ci, et
c) contient une phase amorphe aux rayons X qui, par rapport à la masse totale de mélange pulvérulent, représente au total de 0,1 à 65% en poids en plus de la phase cristalline principale, et sachant que la poudre s'obtient en mélangeant des substances appropriées pour une formation de mélange vitrifiable, 30 - 55% en poids de CaO, 35 - 50% en poids de P₂O₅, 1 - 20% en poids de Na₂O, 0,5 - 20% en poids de K₂O et 0,1 - 5% en poids de MgO ainsi que, le cas échéant, jusqu'à 5% en poids de SiO₂, en homogénéisant et en séchant le mélange vitrifiable et en le soumettant à un traitement thermique par paliers de chaque fois 1-2h à 350-450°C, 750-850°C et 950-1050°C, en faisant fondre le mélange à 1550 à 1650°C, en le maintenant à la température de fusion de 10 à 60 minutes et enfin en le refroidissant spontanément ou à température régulée puis en le broyant.

2. Mélange pulvérulent selon la revendication 1, **caractérisé en ce que** le mélange contient 0,1 - 15% en poids, de préférence 0,5 - 4% en poids de phosphates en chaîne, choisis parmi NaPO₃, KPO₃, des cristaux mixtes de ceux-ci et des mélanges de ceux-ci, présentant des groupes Q₂ lors des mesures de RMN ³¹P.

3. Mélange pulvérulent selon la revendication 1, **caractérisé en ce que** la proportion d'orthophosphates se situe dans la plage de 40 à 95% en poids, de préférence de 50 à 90% en poids.

4. Mélange pulvérulent selon la revendication 1, **caractérisé en ce que** la proportion de diphosphates se situe dans la plage de 1 à 22% en poids, de préférence dans la plage de 5 à 22% en poids.

5. Mélange pulvérulent selon la revendication 1, **caractérisé en ce que**, à l'état fondu ou broyé, il est constitué de en % en poids:
| | | | |
|---|---|---|---|
| 30 | à | 55 | P₂O₅ |
| 25 | à | 50 | CaO |
| 1 | à | 20 | Na₂O |
| 0, 5 | à | 20 | K₂O |
| 0,1 | à | 13 | MgO |
| 0,0 | à | 10 | SiO₂ |
sachant que MgO ou SiO₂ ou un mélange de ceux-ci atteignent au minimum 1% en poids, et des phases cristallines correspondantes.

6. Mélange pulvérulent selon la revendication 1, **caractérisé en ce qu'**il contient jusqu'à 10% d'une phase silicatée.

7. Mélange pulvérulent selon l'une des revendications 1 à 3, **caractérisé en ce qu'**il contient, en plus de la poudre obtenue par le biais d'une masse fondue, jusqu'à 30% en poids en plus d'alpha-triphosphate de calcium, béta-triphosphate de calcium ou des mélanges de ceux-ci.

8. Mélange pulvérulent selon les revendications 1 à 7, **caractérisé en ce qu'**il contient en outre une substance active choisie dans le groupe comprenant des antibiotiques, d'autres substances pharmaceutiques, des désinfectants, des bactériostatiques et des mélanges de ceux-ci.

9. Mélange pulvérulent selon les revendications 1 à 8, **caractérisé en ce qu'**il se présente sous la forme d'une solution aqueuse, d'une suspension ou d'une pâte.

10. Mélange pulvérulent selon la revendication 1, **caractérisé en ce que**, dans les cristaux mixtes, l'élément Ca est remplacé par Mg jusqu'à une proportion de 20% en poids par rapport à la masse du mélange pulvérulent.

11. Mélange pulvérulent selon les revendications 1 à 10, **caractérisé en ce qu'**il se présente sous la forme d'un kit à deux composants sachant que la poudre constitue un composant et la phase aqueuse l'autre composant.

12. Emploi du mélange pulvérulent selon l'une des revendications 1-11 pour la fabrication de biociments résorbables au phosphate de calcium contenant, après durcissement du ciment, moins de 20% en poids d'hydroxylapatite et/ou hydroxylapatite précipitée, en plus des différentes phases cristallines principales de départ.

13. Emploi selon la revendication 12 par mélange d'un mélange pulvérulent selon la revendication 1 avec de l'eau pure ou des solutions aqueuses.

14. Emploi selon la revendication 13, **caractérisé en ce que** le processus de prise avec les solutions aqueuses se fait sous ajout de cations, notamment sodium et potassium, ainsi que d'anions, notamment chlorure.

15. Emploi selon la revendication 14, **caractérisé en ce que** des promoteurs de cohésion et/ou des accélérateurs de prise sont ajoutés à la solution aqueuse.

16. Emploi selon la revendication 15, **caractérisé en ce que** des composés des groupes des hydroxyéthylamidon, amidon soluble, cyclodextrine, alginates, sulfates de dextrane, Polyvinylpyrrolidone et/ou acide hyaluronique sont choisis comme promoteurs de cohésion et que du dihydrogénophosphate de sodium est ajouté à la solution comme accélérateur de prise.

17. Emploi selon la revendication 13, **caractérisé en ce que** le rapport de mélange entre la solution aqueuse et la poudre de ciment est de 0,15 à 0,4 ml/g, de préférence de 0,18 à 0,23 ml/g.

18. Emploi selon la revendication 12 d'un mélange sous forme d'une solution aqueuse, d'une suspension ou d'une pâte pour fabriquer un matériau substitutif des os biodégradable pouvant être employé aussi bien in vivo pour le comblement de lacunes, que in vitro pour la culture cellulaire en ingénierie des tissus.

19. Implant biodégradable à porosité ouverte ou fermée fabriqué à partir d'un mélange sous forme de solution aqueuse, suspension ou pâte, à durcissement ex vivo, selon la revendication 1.
